# EUROPÄISCHE PATENTANMELDUNG

(11) **EP 4 527 364 A1**
(43) Veröffentlichungstag der Anmeldung: **26.03.2025**
(21) Anmeldenummer: 23198991.4
(22) Anmeldetag: 22.09.2023
(51) Int. Cl.: A61F 9/008

(54) **VERFAHREN ZUM AUSBILDEN EINES VORHERSAGEMODELLS, VERFAHREN ZUR ERMITTLUNG VON LASER-BESTRAHLUNGSPARAMETERN SOWIE VORRICHTUNG ZUR NETZHAUTBEHANDLUNG**

(71) Anmelder: OD-OS MacuTherm GmbH, 14513 Teltow (DE)
(72) Erfinder: Charsooghi, Mohammad Avalin, 14129 Berlin (DE); Bengs, Ulrich, 12053 Berlin (DE); Amthor, Kay-Uwe, 14513 Teltow (DE)
(74) Vertreter: Pfenning, Meinig & Partner mbB

(57) **Zusammenfassung**

Die Erfindung bezieht sich auf ein Verfahren zum Ausbilden eines Vorhersagemodells (1, 2), das aktuellen Zustandsparametern (3a, 3b, 3c, 4a, 4b, 4c) eines Patienten (5) einen oder mehrere Laser-Bestrahlungsparameter (10a, 10b, 10c) für eine Netzhautbehandlung zuordnet, wobei als Trainingsdaten für eine Mehrzahl von Patienten (9a, 9b, 9c) jeweils einerseits als Trainings-Eingangsparameter wenigstens eine durch ein bildgebendes Verfahren gewonnene Netzhautaufnahme (7a, 7b, 7c) des Patienten, und andererseits als Trainings-Ergebnisparameter ein Schwellwert für den/die Laser-Bestrahlungsparameter (8a, 8b, 8c), bei dessen/deren Überschreitung bleibende schädigende Gewebeveränderungen eintreten, verwendet werden. Weiter bezieht sich die Erfindung auf ein solches Vorhersagemodell und seine Verwendung bei der Netzhautbestrahlung von Patienten. Insbesondere kann zusätzlich als Trainings-Eingangsparameter wenigstens ein unabhängig von der Netzhaut des Patienten ermittelter Parameter (6a, 6b, 6c), insbesondere ein Hautparameter, ein Haarparameter, ein Irisparameter, das Alter des Patienten, eine physiologisch wirksame Lebensgewohnheit oder eine Vorerkrankung verwendet werden.

## Beschreibung

Die Erfindung liegt auf dem Gebiet der Medizintechnik und der Messtechnik sowie der Lasertechnik und der Datenverarbeitung. Sie ist mit besonderem Vorteil bei der Behandlung der Netzhaut von Patienten bei verschiedenen Krankheiten oder altersbedingten Entwicklungen einsetzbar.

Bei der Laserbehandlung der Netzhaut ist es wünschenswert, die Bestrahlungsintensität geeignet zu wählen und zu steuern, um einerseits einen physiologisch nützlichen Effekt zu erzielen, andererseits aber unterhalb einer Wirksamkeitsschwelle zu bleiben, bei der ernsthafte und nicht wünschenswerte Veränderungen der Netzhaut erzeugt werden. Diese Schwelle ist außer von anderen Randbedingungen wie zum Beispiel den Laserparametern wesentlich auch von physiologischen Eigenschaften der individuellen Netzhaut eines Patienten abhängig, wie beispielsweise von der Pigmentierungsstärke. Es sind Bestrebungen bekannt, physiologische Parameter eines zu behandelnden Patienten individuell zu erfassen, um eine optimierte Netzhautbehandlung durchführen zu können. So ist beispielsweise aus dem Fachartikel: Ivanova, E.V. and Volodin, P.L.: "Development of the selective micropulse individual retinal therapy depends on age and type of Fitzpatrick scale", Graefes Arch. Clin. Exp. Ophtalmol. (2022), bekannt, bei einem Patienten das Alter und einen Hauttyp nach der sogenannten Fitzpatrick- Skala zu ermitteln, wobei dem Ansatz die Annahme zugrunde liegt, dass diese Parameter einen Bezug zu Eigenschaften der Netzhaut aufweisen.

Vor dem Hintergrund des Standes der Technik liegt der vorliegenden Erfindung die Aufgabe zugrunde, ein Verfahren und eine Vorrichtung zur Netzhautbehandlung zu schaffen, die mit einem möglichst geringen Aufwand die Erfassung der notwendigen Parameter eines Patienten erlauben, die eine zuverlässig nützliche und ungefährliche Behandlung der Netzhaut ermöglichen.

Die Aufgabe wird mit der Erfindung durch ein Verfahren zum Ausbilden eines Vorhersagemodells, ein Verfahren zur Ermittlung von Laser-Bestrahlungsparametern sowie eine Einrichtung zur Netzhautbehandlung von Patienten mittels Laserbestrahlung gelöst. Ferner werden vorteilhafte Ausgestaltungen dieser Aspekte angegeben.

Somit bezieht sich die Erfindung unter anderem auf ein Verfahren zum Ausbilden eines Vorhersagemodells, insbesondere zum Trainieren eines neuronalen Netzes oder eines Algorithmus, oder zur Gewinnung von Daten für eine Regressionsanalyse zur Bildung einer Zuordnungsfunktion, wobei das Vorhersagemodell, insbesondere das neuronale Netz, der Algorithmus oder die Zuordnungsfunkton, aktuellen Zustandsparametern eines Patienten einen oder mehrere Laser-Bestrahlungsparameter für eine Netzhautbehandlung zuordnet, wobei als Trainingsdaten für eine Mehrzahl von Patienten jeweils einerseits als Trainings-Eingangsparameter wenigstens eine durch ein bildgebendes Verfahren gewonnene Netzhautaufnahme des Patienten, und andererseits als Trainings-Ergebnisparameter ein Schwellwert für den/die Laser-Bestrahlungsparameter, bei dessen/deren Überschreitung bleibende schädigende Gewebeveränderungen eintreten, verwendet werden, und wobei insbesondere zusätzlich als Trainings-Eingangsparameter wenigstens ein unabhängig von der Netzhaut des Patienten ermittelter Parameter, insbesondere ein Hautparameter, ein Haarparameter, ein Irisparameter, das Alter des Patienten, eine physiologisch wirksame Lebensgewohnheit oder eine Vorerkrankung, verwendet wird.

Eine Netzhautaufnahme für sich oder eine Gruppe von verschiedenen Netzhautaufnahmen mit teilweise überlappendem Informationsgehalt erlaubt bereits die Ableitung einer Vielzahl von Informationen, die mit dem jeweils ermittelten Schwellwert durch das Vorhersagemodell verknüpft werden.

Als Hautparameter, Haarparameter und Irisparameter kann dabei eine Pigmentierungsstärke oder-Dichte verwendet werden, weil diese in gewissem Umfang mit der Pigmentierungsstärke der Netzhaut korreliert. Die Pigmentierungsstärke kann dabei beispielsweise mittels einer Reflektivitätsmessung ermittelt werden. Jedoch kann auch der Hautparameter ebenso wie der Haarparameter oder der Irisparameter durch eine Farbmessung bestimmt werden, indem beispielweise eine fotografische Aufnahme, insbesondere eine Farbaufnahme oder IR-Aufnahme oder Live-Ansicht analysiert wird und die Farbe in einem Farbraum, beispielweise im RGB- oder HSV-Farbraum, anhand der Intensitäten in den jeweiligen Farbkanälen analysiert wird.

Eine physiologisch wirksame Lebensgewohnheit kann beispielsweise die Gewohnheit, zu rauchen, regelmäßiger Alkoholgenuss oder anderer Drogengebrauch oder Übergewicht sein, während als Vorerkrankung beispielsweise Diabetes oder hoher Blutdruck in Frage kommt.

Die optionale Zusammenstellung aus einem unabhängig von der Netzhaut des Patienten ermittelten Parameter des Patienten und wenigstens einer Netzhautaufnahme als Trainings- Eingangsparameter erlaubt eine objektive und dabei schnelle und einfache Datenerhebung. Die Nutzung einer Netzhautaufnahme für sich kann zudem erlauben, beispielsweise Verletzungen der Netzhaut oder Veränderungen im Vergleich zu einem früheren Zustand zu detektieren und zu behandeln oder auch bestimmte Patienten- Datensätze wegen bestimmter untypischer Erscheinungsformen vom Training auszuschließen.

Als Trainings-Ergebnisparameter wird jeweils zu den spezifischen Trainings-Eingangsparametern eines Patienten ein Schwellwert für den/die Laser-Bestrahlungsparameter verwendet, bei dessen/deren Überschreitung bleibende schädigende Gewebeveränderungen eintreten. Zur Ermittlung der Schwellwerte für jede einzelne Netzhaut, die Trainingsdaten liefert, wird die Netzhaut des Patienten an verschiedenen Stellen mit verschiedenen, unterschiedlich intensiven Titrationsschüssen behandelt, das heißt mit Laserbehandlungen unterschiedlicher Intensität. Danach wird durch eine Bildanalyse ermittelt, bei welcher Intensität der Laserbehandlung schädliche Wirkungen in Form von sichtbaren Gewebeveränderungen feststellbar sind. Dazu wird jede Netzhaut, die Trainingsdaten liefert, nach einer oder mehreren verschieden intensiven Laserbehandlungen mittels eines oder mehrerer bildgebender Verfahren analysiert und es wird ermittelt, welches die geringste Laserintensität ist, bei der bereits eine Gewebeschädigung visuell nachweisbar ist. Dieser Wert der Laserintensität kann als Schwellwert festgelegt werden. Dabei können zum visuellen Nachweis von Gewebeschädigungen für jede Netzhaut kumulativ mehrere verschiedene bildgebende Verfahren, auch nach unterschiedlichen Wartezeiten nach der Behandlung, eingesetzt werden, wobei jeweils die in einem der Verfahren nachweisbare unterste Laserintensitätsschwelle, bei der mit einem der Verfahren eine Schädigung ermittelt werden kann, als ermittelte Behandlungsschwelle übernommen werden kann.

Sowohl die im Rahmen der Trainingsdaten bestimmten Schwellwerte als auch der jeweils durch das Vorhersagemodell ermittelte Schwellwert oder die ermittelten Bestrahlungsparameter können nach dem Modell als ortsunabhängige, jeweils für die Netzhaut eines Patienten insgesamt geltende Größen vorliegen und verarbeitet werden. Dadurch ergibt sich eine einfache Handhabung, wobei jedoch bestimmte Sicherheitsgrenzen eingehalten werden. Eine ortsabhängige Gestaltung kann sich aber bei der Behandlung durch ortsabhängige Korrekturgrößen aufgrund eines Behandlungsnah erfassten Fundusbildes ergeben. Grundsätzlich soll jedoch das Vorhersagemodell nicht auf ortsunabhängige Größen beschränkt werden, so dass auch Schwellwerte für die Laserbehandlung für bestimmte Regionen der Netzhaut ermittelt werden können.

Bei der Anwendung des Vorhersagemodells im Rahmen der konkreten Ermittlung von Laser-Bestrahlungsparametern für einen Patienten aus aktuell erfassten Zustandsparametern kann grundsätzlich vorgesehen sein, dass bei dem zu behandelnden Patienten dieselbe Auswahl oder eine ähnliche Auswahl von Eingangsparametern erfasst wird, wie die bei den Trainingsdaten erfassten Trainingseingangsparameter. Die Zuordnung von Bestrahlungsparametern profitiert dann von einer Vielzahl von Erfahrungswerten mit gut untersuchten Patienten.

Ein besonderer Vorteil des erfindungsgemäßen Vorhersagemodells kann jedoch darin liegen, dass vor der konkreten Behandlung eines Patienten deutlich weniger Eingangsdaten erhoben werden als für das Training des Modells, um Zeit, Aufwand und Kosten zu sparen. Ein weiterer Vorteil des Modells kann nämlich darin bestehen, dass es die bestehenden Korrelationen zwischen den teilweise redundanten oder bezüglich ihres Informationsgehaltes erhobenen Trainingsdaten ermittelt und damit im konkreten Anwendungsfall die bei einem zu behandelnden Patienten nicht erhobenen Daten teilweise ersetzen oder überflüssig machen kann. Dies gilt besonders für die Aufnahmen der Netzhaut, die teilweise aufwändig sind. Es ist vorteilhaft, wenn man vor der konkreten Behandlung für die Erfassung aktueller Zustandsdaten eines Patienten mit einer einzigen Aufnahme oder wenigen Aufnahmen auskommt, wobei es besonders vorteilhaft sein kann, wenn eine einzige technische Variante der Netzhautaufnahmen (also zum Beispiel Fundus- Farbfotografie oder IR- Fotografie) ausreicht und gegebenenfalls nur mit einer einzigen Aufnahmeart unter verschiedenen Bedingungen Aufnahmen gemacht werden müssen. Dies erspart dem Patienten und dem behandelnden Arzt Zeit und Aufwand. Unter verschiedenen Bedingungen einer Aufnahmeart kann beispielsweise verstanden werden, dass mehrere Aufnahmen bei verschiedenen Beleuchtungsintensitäten oder verschiedenen beleuchtungsfarben/Wellenlängen gemacht werden.

Die Aussage, dass das Vorhersagemodell aktuellen Zustandsparametern eines Patienten einen oder mehrere Laser-Bestrahlungsparameter für eine Netzhautbehandlung zuordnet, kann bedeuten, dass das Vorhersagemodell jeweils mehrere Laser-Bestrahlungsparameter oder Sätze von Laser-Bestrahlungsparametern für jeweils verschiedene Behandlungsmethoden oder Behandlungsziele angibt/zuordnet oder verschiedene Laser-Bestrahlungsparametersätze, die verschiedene Wahrscheinlichkeiten oder Risiko-Wahrscheinlichkeiten einer Gewebeschädigung entsprechen.

Beispielsweise kann durch das Vorhersagemodell ein Satz von Laser-Bestrahlungsparametern für eine sicher unter der Schädigungsschwelle bleibende Behandlung zugeordnet werden und ein zweiter Satz von Laser-Bestrahlungsparametern für eine Behandlung, bei der eine Gewebekoagulation/teilweise Gewebeschädigung in Kauf genommen wird. In demselben Sinn kann auch die Einrichtung zur Netzhautbehandlung für solche unterschiedlichen Behandlungsansätze eingerichtet sein.

Es soll zudem festgestellt werden, dass im Rahmend es vorliegenden Textes unter einer Laserbehandlung oder -Bestrahlung jeweils neben der Behandlung/Bestrahlung mit einem Laser auch die Behandlung/Bestrahlung mit einer einem Laser gleichwirkenden Lichtquelle, wie beispielsweise einer Leuchtdiode mit ausreichender Leistung und unter einem Behandlungslaser eine gleichwirkende Lichtquelle wie beispielsweise eine Leuchtdiode verstanden werden kann.

Eine besondere Ausgestaltung des Verfahrens kann dabei vorsehen, dass zum Trainieren für jeden Patienten wenigstens zwei Netzhautaufnahmen als Trainings-Eingangsparameter verwendet werden, die zu unterschiedlichen Zeitpunkten, insbesondere vor und nach einer Titration, und/oder mit verschiedenen bildgebenden Verfahren und/oder bei unterschiedlichen technischen Aufnahmebedingungen gewonnen werden.

Dabei kann vorgesehen sein, dass als bildgebende Verfahren für die Ermittlung der Trainings-Eingangsparameter eines oder zwei der folgenden Verfahren verwendet werden:
Fotografische Farbbildaufnahme, fotografische Infrarotaufnahme (IR-Fotografie), OCT-B-scan (Tiefenschnittbild einer mit optischer Kohärenztomografie erstellten Aufnahme), BAF (Blue Autofluorescence, Fluoreszenzaufnahme ohne zusätzlichen Farbstoff, Anregungswellenlänge 488nm), Scanning laser ophtalmoscopy, OCT in Verbindung mit Scanning laser ophtalmoscopy), FAG (Fluorescence Angiography, Fluoreszenzaufnahme mit Farbstoff Fluorescein, Anregungswellenlänge 488nm).

Es kann somit beispielsweise vorgesehen werden, dass eine Netzhautaufnahme mittels Fundus-Farbfotografie mit einer IR-Fotografie kombiniert wird oder mit einer OCT- B-Aufnahme oder mit einer blue-Autofluoreszenzaufnahme (BAF) oder mit einer Scanning-Laser- Ophtalmoskopie- Aufnahme (SLO), oder es können mehrere Fundus- Farbfotografien bei verschiedenen Beleuchtungsintensitäten oder verschiedenen Beleuchtungsspektren miteinander kombiniert werden oder eine IR- Fotografie mit eine OCT- Aufnahme oder einer SLO. Es kann auch eine OCT-Aufnahme mit einer SLO oder eine von diesen mit BAF kombiniert werden. Jede der genannten Methoden kann auch beispielsweise mit einer FAG- Aufnahme kombiniert werden. Es können auch mehrere Fundus- Farbfotografien oder IR- Aufnahmen unter verschiedenen Aufnahmebedingungen miteinander kombiniert werden oder mehrere Fundus- Farbfotografien oder IR-Aufnahmen unter verschiedenen Aufnahmebedingungen mit einem anderen der genannten Verfahren.

Auch die Kombination von drei oder mehr der genannten Verfahren ist selbstverständlich zur Erzeugung von Trainingsdaten möglich.

Durch diesen Ansatz werden nicht nur mehr Daten für das Training oder die Ermittlung von Daten für eine Regressionsanalyse zur Verfügung gestellt, sondern es werden auch die Korrelationen zwischen den verschiedenen Arten der Netzhautaufnahmen und die Redundanzen/Überlappungen des Informationsgehaltes ermittelt und in das Vorhersagemodell eingespeist. Ergibt sich beispielsweise für zwei verschiedene Aufnahmearten grundsätzlich dieselbe aus diesen ableitbare Aussage, so kann bei der Anwendung des Vorhersagemodells am konkreten Behandlungsfall die Erhebung von aktuellen Patientendaten ohne substanzielle Qualitätsverluste auf eine der beiden Aufnahmearten beschränkt oder wenigstens auf eine geringere Anzahl von Aufnahmen beschränkt werden, als für die Erstellung von Trainingsdaten verwendet wurden.

Im Übrigen kann im Anwendungsfall eher eine oder eine Gruppe der weniger aufwändigen Methoden zur Erstellung von Netzhautaufnahmen zur Ermittlung von aktuellen Zustandsdaten eines Patienten verwendet werden, das heißt, beispielsweise eher eine weniger Kosten- oder zeitaufwändige Methode oder eine Methode, die sich weniger fehleranfällig auch ohne Fachpersonal oder zumindest mit angelerntem Personal durchführen lässt.

Bei einem Verfahren der beschriebenen Art kann weiter vorgesehen sein, dass der jeweilige Schwellwert für den/die Laser-Bestrahlungsparameter für jeden Patienten durch eine Reihe von Titrationsschüssen auf die Netzhaut des Patienten mit verschiedenen Laser-Bestrahlungsparametern und einen nachfolgenden Nachweis von Gewebeveränderungen anhand von jeweils mehreren, durch verschiedene bildgebende Verfahren gewonnenen Netzhautabbildungen ermittelt wird, wobei insbesondere jeweils der geringste mit einem der bildgebenden Verfahren ermittelte Schwellwert als Trainings- Ergebnisparameter für das Ausbilden des Vorhersagemodells, insbesondere das Training eines neuronalen Netzes, verwendet wird.

Bei der Erstellung der Trainingsdaten kann auf diese Weise die Schwellwert für den/die Laser-Bestrahlungsparameter für jeden Patienten, bei dessen Erreichen oder Überschreitung eine bleibende Gewebeschädigung eintritt, mit erhöhter Sicherheit und Sensitivität sowie Zuverlässigkeit ermittelt werden. Es kann nicht ausgeschlossen werden, dass bei einzelnen Gruppen von Patienten bestimmte Nachweisverfahren sensitiver sind als bei anderen Gruppen. Zudem können bestimmte Verfahren nach einer bestimmten Wartezeit nach den Titrationsschüssen gegenüber anderen Verfahren an Sensitivität beim Nachweis von Gewebeveränderungen gewinnen.

Die Auswertung der Netzhautaufnahmen kann in jedem der verwendeten Verfahren beispielsweise maschinell durch eine digitale Bildanalyse erfolgen. Damit ergeben sich objektivierte Maßstäbe.

Zur konkreten Ermittlung der Schwellwerte bei der Gewinnung von Trainingsdaten kann vorgesehen sein, dass der jeweilige Schwellwert für den/die Laser-Bestrahlungsparameter für jeden Patienten durch eine Reihe von Titrationsschüssen auf die Netzhaut des Patienten mit verschiedenen Laser-Bestrahlungsparametern und einen nachfolgenden Nachweis von Gewebeveränderungen anhand von jeweils mehreren, durch verschiedene bildgebende Verfahren gewonnenen Netzhautabbildungen ermittelt wird, wobei als Trainings-Ergebnisparameter für das Ausbilden des Vorhersagemodells, insbesondere das Training eines neuronalen Netzes, für alle untersuchten Patienten der durch dasjenige bildgebende Verfahren ermittelte Schwellwert verwendet wird, welches bei den meisten untersuchten Patienten den geringsten Schwellwert anzeigt.

Konkret kann in einer Realisierung des Verfahrens vorgesehen sein, dass der jeweilige Schwellwert für den/die Laser-Bestrahlungsparameter für jeden Patienten als Trainings-Ergebnisparameter durch eine Reihe von Titrationsschüssen auf die Netzhaut des Patienten mit verschiedenen Laser-Bestrahlungsparametern und einen nachfolgenden Nachweis von Gewebeveränderungen anhand der optischen Kohärenztomografie in Verbindung mit IR-scanning Laser-Ophtalmoskopie ermittelt wird.

Es kann weiter bei dem Verfahren der oben genannten Art vorgesehen sein, dass als Laser-Bestrahlungsparameter, der bei den Titrationsschüssen variiert und dessen Schwellwert als Trainings-Ergebnisparameter ermittelt wird, eine Leistung des Bestrahlungslasers oder eine Leistungsdichte des Laserspots bei ansonsten festgelegten Laser-Bestrahlungsparametern oder eine über die Bestrahlungszeit insgesamt deponierte Energie auf einem Laserspot festgelegter Größe gewählt wird.

Jede dieser Größen kann bei im Übrigen gleichbleibenden Randbedingungen bei der Laserbestrahlung variiert werden, wodurch bei Überschreiten einer Schwelle bleibende Gewebeveränderungen erzeugt werden können. Die Schwelle für Gewebeveränderungen kann durch die beschriebenen Versuchsserien ermittelt werden.

Eine besondere Herausforderung kann bei der Ermittlung der Trainings-Eingangsparameter die möglichst objektivierte Angabe von netzhautunabhängigen Parametern des jeweiligen Patienten darstellen. Diese können einerseits im Rahmen einer Anamnese ermittelt und über eine Eingabeeinrichtung eingegeben werden. Dieses Verfahren eignet sich beispielsweise für die Altersangabe, die Angabe von Krankheiten oder Gewohnheiten. Besonders aussagekräftig sind jedoch die Angaben, die sich auf die Hautparameter, Haarparameter und Irisparameter beziehen. Diese können einerseits durch Einschätzung auf einer Skala erhoben werden, beispielsweise durch Verortung auf der bekannten Fitzpatrick- Skala oder auf jeweils gesonderten Farb- oder Helligkeitsskalen für die einzelnen Parameter. Es sind jedoch auch technische Messverfahren zur Ermittlung der Helligkeit oder Farbe von Haut, Haaren und Iris einsetzbar, beispielsweise durch Farbfotografie oder IR- Fotografie unter definierten Beleuchtungsbedingungen. Dabei kann die Helligkeit oder Farbverteilung in festgelegten Farbkanälen objektiv gemessen und als Parameterwert angegeben werden. Bei der Auswertung von Fotografien kann auch ein trainiertes neuronales Netz für die Auswertung verwendet werden, das mit einer Anzahl an Fotografien und zugeordneten parametrisierten Bewertungen trainiert ist. Das so trainierte neuronale Netz kann auch mit dem oben beschriebenen, trainierten neuronalen Netz identisch sein, das heißt, das oben beschriebene Vorhersagemodell kann zusätzlich auf die Einordnung von Fotografien der Patienten in verschiedene Haut-/Haar/- Iristypen trainiert sein. Somit kann im Rahmen des beschriebenen Verfahrens auch vorgesehen sein, dass ein Hautparameter und/oder ein Haarparameter und/oder ein Irisparameter durch ein optisches Messverfahren ermittelt wird, wobei insbesondere eine Farbe und/oder eine Reflektivität der Haut, des Haars oder der Iris eines Patienten gemessen wird.

Die Erfindung bezieht sich außer auf ein Verfahren zum Ausbilden eines Vorhersagemodells der oben beschriebenen Art auch auf ein Vorhersagemodell, das aktuellen Zustandsparametern eines Patienten einen oder mehrere Laser-Bestrahlungsparameter für eine Netzhautbehandlung zuordnet und das nach dem oben beschriebenen Verfahren, insbesondere durch Trainieren eines neuronalen Netzes oder eine Regressionsanalyse und Bildung einer Zuordnungsfunktion, ausgebildet ist.

Ein solches Vorhersagemodell kann in der Form eines trainierten neuronalen Netzes oder eines Datenverarbeitungsprogramms vorliegen und beispielsweise auf einem Datenträger gespeichert sein. Wird ein solches Datenverarbeitungsprogramm durch einen Prozessor oder eine Datenverarbeitungsanlage durchgeführt, so kann das Verfahren, das sich ergibt, die Zuordnung von Laser- Bestrahlungsparametern für eine Netzhautbehandlung zu aktuellen Zustandsparametern eines Patienten vornehmen.

Die Erfindung bezieht sich zudem auf ein Verfahren zur Ermittlung von Laser-Bestrahlungsparametern zur Behandlung der Netzhaut eines Patienten, bei dem in einem ersten Schritt aktuelle Zustandsparameter des Patienten ermittelt werden und in einem weiteren Schritt den Zustandsparametern durch ein Vorhersagemodell der oben beschriebenen Art ein oder mehrere Laser-Bestrahlungsparameter für eine Netzhautbehandlung zugeordnet werden, wobei die Zustandsparameter eine, zwei oder mehr als zwei jeweils durch ein bildgebendes Verfahren gewonnene Netzhautaufnahmen des Patienten umfassen, wobei im Fall von mehr als einer Netzhautaufnahme die Netzhautaufnahmen mit verschiedenen bildgebenden Verfahren und/oder unter verschiedenen Aufnahmebedingungen gewonnen werden. Die Zuordnung der Laser-Bestrahlungsparameter für eine Netzhautbehandlung zu den aktuellen Zustandsparametern kann durch das oben beschriebene Vorhersagemodell, insbesondere das neuronale Netz durchgeführt werden, das gemäß dem oben beschriebenen Verfahren trainiert worden ist.

Zudem kann bei einem solchen Verfahren vorgesehen sein, dass die Zustandsparameter einen oder mehrere der folgenden Parameter umfassen: einen Hautparameter, einen Haarparameter, einen Irisparameter, das Alter des Patienten, eine physiologisch wirksame Lebensgewohnheit, eine Vorerkrankung.

Die zu ermittelnden Laser-Bestrahlungsparameter können beispielsweise eine Laserleistung, maximale Dauer der Bestrahlung an einem Laserspot, eine Laserleistungsdichte, jeweils potenziell verbunden mit einem duty cycle oder eine aufsummierte, an einem Spot in das Netzhautgewebe durch die Laserintensität eingebrachte Energiemenge sein. Diese Größen sollen gezielt so gewählt werden können, dass bei einer Laserbestrahlung mit diesen Parametern entweder keine bleibende Gewebeschädigung in der Netzhaut erfolgt oder eine genau kalkulierte Gewebeveränderung.

Als bildgebende Verfahren für die Erstellung von aktuellen Netzhautaufnahmen, die als aktuelle Zustandsparameter verwendet werden, können ein oder mehrere der folgenden Verfahren verwendet werden, die grundsätzlich auch bei der Erzeugung von Trainingsdatensätzen zum Trainieren des oben beschriebenen Vorhersagemodells zur Anwendung kommen können:
Fotografische Farbbildaufnahme oder mehrere Farbbildaufnahmen bei mehreren Sätzen von Beleuchtungsbedingungen, beispielsweise Beleuchtungsintensitäten oder -Spektren, fotografische Infrarotaufnahme (IR-Fotografie), ebenfalls gegebenenfalls mehrere Aufnahmen bei unterschiedlichen Beleuchtungsbedingungen, beispielsweise Beleuchtungsintensitäten oder -Spektren, OCT-B-scan (Tiefenschnittbild einer mit optischer Kohärenztomografie erstellten Aufnahme), BAF (Blue Autofluorescence, Fluoreszenzaufnahme ohne zusätzlichen Farbstoff, Anregungswellenlänge 488nm), Scanning laser ophtalmoscopy, OCT+SLO IR (optische Kohärenztomografie in Verbindung mit IR-scanning Laser- Ophtalmoskopie, einem Konfokalen Bildgebungsverfahren im Infrarotbereich), FAG (Fluorescence Angiography, Fluoreszenzaufnahme mit Farbstoff Fluorescein, Anregungswellenlänge 488nm).

Besonders vorteilhaft sind dabei die Aufnahmemethoden, die einfach, schnell und ohne die Hilfe von Fachpersonal sowie mit einfach zu bedienenden, kostengünstigen Apparaten durchführbar sind, wie die Farbbild- und IR- Fotografie. Auch die BAF- Methode ist, da kein Kontrastmittel eingesetzt wird, einfach und schnell durchführbar. Es sind auch beispielsweise jeweils zwei der zuletzt genannten drei Verfahren gut miteinander kombinierbar. Grundsätzlich kann auch eine einzige der oben genannten Netzhautaufnahmen ausreichen, auch für diesen Fall sind die drei zuletzt genannten Methoden besonders aufwandsarm und einfach auswertbar.

Als Hautparameter, Haarparameter und Irisparameter kann auch bei der Ermittlung aktueller Zustandsgrößen eines Patienten eine Pigmentierungsstärke oder -Dichte verwendet werden, weil diese in gewissem Umfang mit der Pigmentierungsstärke der Netzhaut korreliert. Die Pigmentierungsstärke kann dabei beispielsweise mittels einer Reflektivitätsmessung ermittelt werden. Jedoch kann auch der Hautparameter ebenso wie der Haarparameter oder der Irisparameter durch eine Farbmessung bestimmt werden, indem beispielsweise die Intensitäten in mehreren Farbkanälen, beispielsweise des RGB- Systems, erfasst werden.

Dabei kann konkret weiter vorgesehen sein, dass ein Hautparameter und/oder ein Haarparameter und/oder ein Irisparameter des Patienten durch ein optisches Messverfahren ermittelt wird, wobei insbesondere eine Farbe und/oder eine Reflektivität der Haut, des Haars oder der Iris des Patienten gemessen wird und/oder zur Bestimmung der Zustandsparameter jeweils eine fotografische Aufnahme indem beispielweise eine fotografische Aufnahme, insbesondere eine Farbaufnahme oder IR-Aufnahme oder Live- Ansicht der Haut, der Haare oder der Iris des Patienten analysiert wird und die Farbe in einem Farbraum, beispielweise im RGB- oder HSV-Farbraum, anhand der Intensitäten in den jeweiligen Farbkanälen analysiert, beispielsweise durch das Vorhersagemodell ausgewertet wird.

Eine physiologisch wirksame Lebensgewohnheit kann beispielsweise die Gewohnheit, zu rauchen, regelmäßiger Alkoholgenuss oder anderer Drogengebrauch oder Übergewicht sein, während als Vorerkrankung beispielsweise Diabetes, Übergewicht und Bluthochdruck in Frage kommt.

Die Zusammenstellung aus einem unabhängig von der Netzhaut des Patienten ermittelten Parameter und wenigstens einer Netzhautaufnahme als aktuelle Zustandsparameter eines Patienten erlaubt eine objektive und dabei schnelle und einfache Datenerhebung.

Eine weitere Ausgestaltung des oben beschriebenen Verfahrens kann vorsehen, dass aus wenigstens einer Netzhautaufnahme des Patienten Bereiche ermittelt und für eine nachfolgende Laserbehandlung von der Bestrahlung ausgenommen werden, deren Reflektivität von einer Durchschnittsreflektivität der Netzhaut um wenigstens 50% abweicht, insbesondere mehr als 50% höher ist als die Durchschnittsreflektivität der Netzhaut oder 50% geringer als die Durchschnittsreflektivität der Netzhaut.

Es wird dabei von der Annahme ausgegangen, dass die Bereiche der Netzhaut, bei denen die Reflektivität deutlich höher oder geringer ist als in den übrigen Bereichen, die Bereiche sind, die praktisch nicht pigmentiert sind und/oder keine Behandlung benötigen. Es sind diese bei Verwendung der Farb-Fundusfotografie oder der Infrarotfotografie im Besonderen der optische Nervenkopf mit hoher Reflektivität und die Bereiche über den Haupt- Blutgefäßen, die die Netzhaut versorgen, mit deutlich niedrigerer Reflektivität als die übrigen Bereiche der Netzhaut (letztere aufgrund des Strahlung absorbierenden Hämoglobins). Bei Verwendung anderer bildgebender Verfahren kann der Kontrast sich anders darstellen. So erscheinen in einer durch Fluoreszenz-Angiographie gewonnenen Aufnahme die Bereiche der Blutgefäße deutlich, das heißt auch mindestens 50% heller als die übrigen Bereiche der Netzhaut, während bei Verwendung einer Autofluoreszenz diese Bereiche deutlich dunkler als die übrigen Bereiche der Netzhaut erscheinen.

Das beschriebene Verfahren zur Ermittlung von Laser- Bestrahlungsparametern kann auch dadurch ausgestaltet werden, dass aus der Netzhautaufnahme des Patienten die Varianz der Reflektivitätswerte, deren Differenz von der Durchschnittsreflektivität um weniger als 50% differieren, ermittelt und aus der Varianz ein Korrekturwert für die durch das Vorhersagemodellermittelten Laser-Bestrahlungsparameter ermittelt wird und/oder, dass aus der auf dem Nervenkopf ermittelten Reflektivität ein Verlustparameter ermittelt wird, der die Verluste durch Absorption und Streuung von Licht im Glaskörper des Auges beschreibt und dass dieser Verlustparameter bei der Bestimmung der Laserbestrahlungsparameter berücksichtigt wird.

Durch die Eliminierung der Bereiche, deren Durchschnittsreflektivität mehr als 50% mehr beträgt als die der übrigen Bereiche, werden die nicht pigmentierten Bereich der Netzhaut, die weiter oben beschrieben worden sind, aus der Berechnung ausgenommen. Mit der Bestimmung der Varianz der Reflektivität in den verbleibenden Bereichen kann ein Korrekturwert der Laser-Bestrahlungsparameter gewonnen werden, durch den die Sicherheit vor schädigenden Laserwirkungen erhöht wird.

Aus einem Reflektivitätswert, der im Bereich des Nervenkopfes gemessen wird, kann unter Berücksichtigung einer Rest- Absorption der Netzhaut, die nicht von einer Pigmentierung abhängt, der Intensitätsverlust berechnet werden, der auf dem Weg eines Laserstrahls durch den Glaskörper des Auges bis zur Netzhaut und zurück durch den Glaskörper eintritt. Dieser setzt sich additiv zusammen aus den Absorptionsverlusten im Glaskörper und der Streuung, die beide unter anderem auch vom Gesundheitszustand des Auges, insbesondere dem Vorliegen einer Katarakterkrankung abhängen. Bei einem Intensitätsverlust durch diese Effekte kann zur Korrektur eine Erhöhung der Laserintensität bei der Behandlung stattfinden.

Die Erfindung bezieht sich schließlich auch auf eine Einrichtung zur Netzhautbehandlung von Patienten mittels Laserbestrahlung mit wenigstens einem Behandlungslaser sowie wenigstens einer Vorrichtung zur Erstellung einer Netzhautaufnahme und mit einer Verarbeitungseinrichtung mit einem Vorhersagemodell der oben beschriebenen Art, die wenigstens einer aktuell durch die Vorrichtung erstellten Netzhautaufnahme und insbesondere einem zusätzlichen, aktuell ermittelten Zustandsparameter einen oder mehrere Laser-Bestrahlungsparameter zur Steuerung für den Behandlungslaser zuordnet.

Die Einrichtung zur Netzhautbehandlung von Patienten mittels Laserbestrahlung ist in einer an sich bekannten Art ausgeführt und kann bezüglich des benutzten optischen Systems mit einer oder zwei oder mehreren Einrichtungen zur Erstellung einer Netzhautaufnahme baulich verbunden sein. Dadurch kann in einfacher Weise ein gemeinsames Bezugssystem für die zu behandelnde Netzhaut für die Messung und Behandlung und gegebenenfalls auch geschaffen werden. Es ist dadurch auch zuverlässig möglich, bestimmte individuell detektierte Bereiche der Netzhaut wie die primären Blutgefäße und den optischen Nervenkopf von der Bestrahlung auszunehmen. Zudem ist ein solches System auch für eine Beobachtung der Netzhaut während der Behandlung und gegebenenfalls für ein Augentracking, das heißt die Beobachtung und Kompensation von Augenbewegungen während der Behandlung, zu verwenden.

Beispielsweise kann mit dem Behandlungslaser ein System zur Fundus-Farbfotografie und/oder IR -Fotografie und/oder FAG (Fluoreszenz-Angiografie) verbunden sein.

Zudem kann in die Vorrichtung eine Messeinrichtung zur Ermittlung eines Hautparameters, eines Haarparameters oder eines Irisparameters integriert sein. Diese Vorrichtung kann beispielsweise eine Farbbildkamera oder IR- Kamera oder einen Farbbildscanner zur Aufnahme eines Bildes der Haut, des Haars/Haupthaars/der Augenbrauen oder der Iris des Patienten umfassen sowie eine Analyseeinrichtung, die einer Aufnahme einen Zustandsparameter durch Auswertung einer Reflektivität oder einer Farbe zuordnet. Die Kamera oder der Scanner kann beispielsweise in eine Kammer integriert sein, die eine Beleuchtungseinrichtung sowie ein Fenster aufweist, vor dem die Haut oder das Haar oder die Iris des Patienten angeordnet werden kann. Die Beleuchtung kann dabei bezüglich Intensität und/oder des Farbspektrums variabel sein, um durch Differenz- oder Quotientenbildung verschiedener Aufnahmen unter verschiedenen Beleuchtungsbedingungen zusätzliche Informationen zu gewinnen.

Im Folgenden wird die Erfindung anhand von Ausführungsbeispielen in Figuren einer Zeichnung gezeigt und nachfolgend erläutert.

Dabei zeigt
- Figur 1:: eine Darstellung des Trainingsprozesses für ein Vorhersagemodell,
- Figur 2:: eine Darstellung der Nutzung des trainierten Vorhersagemodells zur Ermittlung von Laser- Bestrahlungsparametern, sowie
- Figur 3:: eine weitere Darstellung der Ausbildung des Vorhersagemodells.

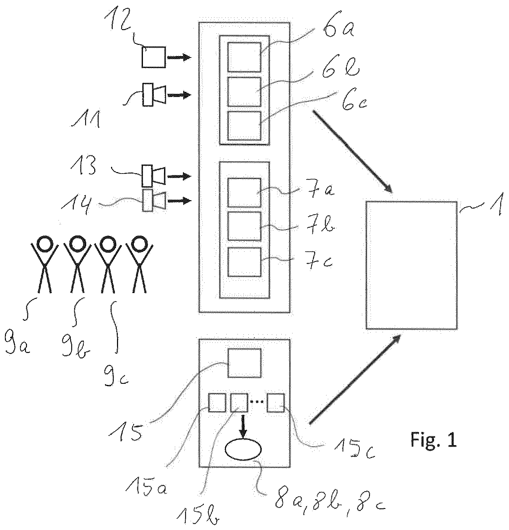

Figur 1 zeigt schematisch einen Prozess zur Ausbildung eines Vorhersagemodells, konkret zum Trainieren eines neuronalen Netzes 1 mit Trainingseingangsdaten 6a, 6b, 6c, 7a, 7b, 7c und Trainings-Ergebnisdaten 8a, 8b, 8c, die jeweils an einer größeren Gruppe von Personen 9a, 9b, 9c ermittelt und dem neuronalen Netz 1 oder einer Einrichtung zur Regressionsanalyse zum Training oder zur Auswertung zur Verfügung gestellt werden.

Als Trainings- Eingangsdaten können zunächst Aufnahmedaten aus einer, zwei oder mehr Aufnahmen 7a, 7b, 7c, insbesondere einer Bildaufnahme, der Netzhaut der jeweiligen Person 9a, 9b, 9c bereitgestellt werden. Als Aufnahmemodus kommen beispielsweise Fundus-Farbfotografien oder IR- Fotografien, jeweils bei einer oder mehreren Beleuchtungssituationen in Frage, wobei eine Beleuchtungssituation durch eine Beleuchtungsintensität, einen Beleuchtungswinkel und durch ein bestimmtes Beleuchtungsspektrum definiert sein kann. Weiter kommen ein OCT-B-scan (Tiefenschnittbild einer mit optischer Kohärenztomografie erstellten Aufnahme), BAF (Blue Autofluorescence, Fluoreszenzaufnahme ohne zusätzlichen Farbstoff, Anregungswellenlänge 488nm), OCT+SLO IR (optische Kohärenztomografie in Verbindung mit IR-scanning Laser- Ophtalmoskopie, einem konfokalen Bildgebungsverfahren im Infrarotbereich), FAG (Fluorescence Angiography, Fluoreszenzaufnahme mit Farbstoff Fluorescein, Anregungswellenlänge 488nm) in Frage. In der Figur 1 sind die Aufnahmeeinrichtungen für verschiedene Aufnahmen symbolisch durch die Kameras 13, 14 repräsentiert, die so zueinander ausgerichtet sind, dass Referenzpunkte der verschiedenen Bilder zueinander in Beziehung gesetzt werden können.

Zusätzlich können noch als weitere Trainings-Eingangsparameter 6a, 6b, 6c Haut- und/oder Haar- und/oder Irisparameter sowie weitere gesundheitsrelevante Parameter wie das Alter, bestimmte Lebensgewohnheiten und Vorerkrankungen ermittelt und dem neuronalen Netz oder einer Regressionsanalyse als Trainings-Eingangsparameter zur Verfügung gestellt werden. Die Haut- und/oder Haar- und/oder Irisparameter können mittels einer Messeinrichtung 11, beispielsweise einer Farbbildkamera, einer IR-Kamera oder einer Hyperspektralkamera, jeweils mit Auswerteeinrichtung ermittelt werden. Diese und zusätzliche Parameter können auch mit einer Dateneingabeeinrichtung 12, beispielsweise einer Tastatur oder mit einer Spracherkennungseinrichtung mittels Mikrofon eingegeben werden.

Die genannten Trainingseingangsparameter liefern dem Vorhersagemodell zunächst die Datenbasis für eine umfassende Zustandsbeschreibung der jeweiligen Person.

Mit den genannten Daten/Trainings-Eingangsparametern werden die ermittelten Schwelldaten für die Laser-Bestrahlungsparameter der jeweiligen individuellen Person 9a, 9b, 9c verknüpft, die die Trainings-Ergebnisparameter 8a, 8b, 8c bilden. Diese Schwelldaten werden für jede Person dadurch ermittelt, dass auf Ihre Netzhaut mittels eines Lasers Titrationsschüsse mit verschiedenen Laser-Bestrahlungsparametern, das heißt mit verschiedenen Intensitäten, abgegeben werden. Individuell wird für jede Person ausgewertet, welche der Titrationsschüsse bleibende Gewebeveränderungen hervorgerufen haben und dadurch wird die Schwelle oder Laserschwelle für jede Person 9a, 9b, 9c individuell bestimmt und als Trainings-Ergebnisparameter dem Vorhersagemodell zur Verfügung gestellt.

Zum Nachweis der Gewebeveränderungen können durch eine Aufnahmeeinrichtung 15 verschiedene Aufnahmemodi 15a, 15b, 15c zur Aufnahme der Netzhaut der jeweiligen Person genutzt werden. Dazu kommen grundsätzlich alle oben genannten Aufnahmemodi, auch mehrere in Kombination, in Frage. Als besonders sensitiv hat sich die Blue Autofluorescence- Methode herausgestellt. Diese kann mit einer oder mehreren der anderen genannten Methoden kombiniert werden, um in jedem einzelnen Fall Gewebeveränderungen möglichst sensitiv zu erkennen. Die Ergebnisse der Titrationsschüsse können durch Experten in verschiedenen Aufnahmen, die durch verschiedene Aufnahmemodi erstellt worden sind, beurteilt werden, wobei beispielsweise als Standard jeweils der Schwellwert herangezogen wird, der in irgendeiner der Aufnahmen bei dem Titrationsschuss mit der geringsten Intensität zu einer erkennbaren Gewebeveränderung geführt hat. Dabei kann mit der Untersuchung der Netzhaut auch eine gewisse Zeit, beispielsweise eine oder mehrere Wochen nach den Titrationsschüssen abgewartet werden.

Grundsätzlich kann aber auch ermittelt werden, welche der Aufnahmemodi bei den meisten Versuchspersonen der sensitivste ist und dieser Modus kann dann für die Beurteilung aller Personen herangezogen werden. Die Erkennung von Gewebeveränderungen kann auch durch eine digitale Bildverarbeitung erfolgen.

Anstelle eines neuronalen Netzes 1 kann auch mit den ermittelten und gesammelten Daten eine Regressionsanalyse durchgeführt werden und es können die Einflüsse von einzelnen Merkmalen der Trainings-Eingangsparameter auf die Laserschwelle bestimmt und gewichtet werden. Mit der Regressionsanalyse kann ein Algorithmus trainiert/ausgebildet werden, der dann als Vorhersagemodell dient.

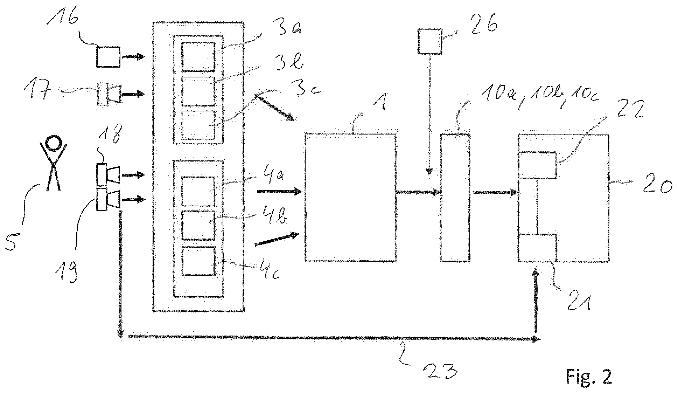

In der Figur 2 ist schematisch ein System und Verfahren zur Verwendung des Vorhersagemodells 1 bei der konkreten Behandlung von Patienten 5 gezeigt. Der Patient 5 nutzt dazu eine Aufnahmeeinrichtung 18, 19, die eine Aufnahme 4a, 4b, 4c von seiner Netzhaut oder Teilen der Netzhaut erstellt.

Potenziell erfassen weitere Aufnahmeeinrichtungen 16 und/oder 17, beispielsweise in Form einer Farbbildkamera, IR-kamera oder Hyperspektralkamera die Reflektivität, Helligkeit oder Farbe von Haut, Haaren oder Iris des Patienten, die Beispiele für unabhängig von der Netzhaut des Patienten ermittelte Zustandsdaten 3a, 3b, 3c darstellen. Im einfachsten Fall kann dies auch durch einen lichtempfindlichen Sensor und eine Beleuchtungseinrichtung geschehen, die in einer gemeinsamen Messkammer angeordnet sind, wobei die Kammer ein Fenster aufweist und Haut, Haare oder das Auge des Patienten vor dem Fenster positioniert werden.

Die Aufnahmeeinrichtung kann beispielsweise nur eine einzige technische Einrichtung zur Aufnahme eines einzigen Aufnahmemodus, beispielsweise aus der oben angeführten Liste, aufweisen, beispielsweise eine Fundus-Farb- oder Infrarotkamera oder eine Einrichtung zur Blue-Autofluorescence-Aufnahme. Grundsätzlich kann die Aufnahmeeinrichtung auch nur eine Kamera zur Erfassung verschiedener Aufnahmemodi unter unterschiedlichen Beleuchtungsbedingungen aufweisen, da auch in diesem Fall der technische Aufwand sehr gering bleibt. Es können aber auch zwei oder mehr Kameras zur Erfassung unterschiedlicher Aufnahmemodi kombiniert werden. Grundsätzlich kann vorgesehen sein, dass bei der Erfassung der aktuellen Zustandsdaten eines Patienten 5 vor seiner Behandlung Aufnahmen mit weniger verschiedenen Aufnahmemodi gemacht und für die Analyse verwendet werden als für das Trainieren des verwendeten Vorhersagemodells 1, 2.

Die mit den lokalen Einrichtungen 16, 17, 18, 19 erfassten aktuellen Zustandsdaten können am Ort der Behandlung weiterverarbeitet werden, jedoch können sie auch mittels eines verteilten Computernetzes, beispielsweise mit einem entfernten Server, weiter verarbeitet werden, wobei auch das Vorhersagemodell 1, 2 am Ort der Behandlung oder von diesem entfernt angeordnet sein kann. Den mit den lokalen Einrichtungen 16, 17, 18, 19 erfassten Daten/aktuellen Zustandsdaten des Patienten 5 werden durch das Vorhersagemodell 1, 2 ein oder mehrere Laser-Bestrahlungsparameter zugeordnet, beispielsweise Laserintensitäten für eine gewebeverändernde Behandlung sowie eine Obergrenze/Schwelle für die Laserintensität, bei der Gewebeveränderungen sicher vermieden werden. Unter der Laserintensität werden in diesem Zusammenhang alle Parameter des Lasers und der Strahlführung verstanden, die auf eine potenzielle Gewebeschädigung Einfluss haben, wie beispielsweise die Laserleistung, die Leistungsdichte, der duty cycle, die Verweilzeit des Lasers auf einem Spot, die gesamte in einen Laserspot eingetragene Energie.

Grundsätzlich kann die ermittelte Laserschwelle für die gesamte zu behandelnde Netzhaut einheitlich sein, sie kann jedoch auch noch eine Ortsabhängigkeit aufweisen, beispielsweise den Nervenkopf und die primären Blutgefäße von der Behandlung ausschließen. Zudem können aufgrund der verwendeten Netzhautaufnahme auch bestimmte singuläre Stellen der Netzhaut ausgenommen werden.

Es kann auch vorgesehen sein, dass mit der aktuellen Netzhautaufnahme die Varianz/Variabilität der Pigmentierung/Absorptionsstärke der Netzhaut ermittelt wird, das heißt der Umfang von Abweichungen vom Durchschnitt der Pigmentierung/Absorptionsstärke von einem ermittelten Durchschnittswert und dass aus der Varianz ein Korrekturwert für die Laser-Bestrahlungsparameter ermittelt wird, der die Laserintensität bei einer hohen Varianz verringert, um die Orte hoher Absorption auf der Netzhaut nicht zu schädigen, falls die Netzhaut insgesamt mit nicht ortsabhängigen Laser-Bestrahlungsparametern behandelt wird. Bei der Ermittlung des Durchschnittswertes der Pigmentierung/Absorption der Netzhaut können die Orte des Nervenkopfes und der primären Blutgefäße ausgenommen werden.

Es ist zudem auch möglich, aus der gemessenen und aus der aktuellen Aufnahme ermittelten Reflektivität an singulären Stellen der Netzhaut, wie beispielsweise dem Nervenknoten, einen Verlustfaktor zu bestimmen, der die Absorption und Streuung der Laserstrahlung im Glaskörper des Auges repräsentiert. Bei einem nachweisbaren Verlustfaktor kann die Laserintensität bei einer nicht ortsabhängigen Behandlung zu einem Korrekturfaktor der Laser-Bestrahlungsintensität führen, der größer als 1 ist und beispielsweise zwischen 1 und 1,5 liegt.

Wie in der Figur 2 ersichtlich ist, werden die mit den lokalen Einrichtungen 16, 17, 18, 19 erfassten Daten/aktuellen Zustandsdaten des Patienten 5 dem Vorhersagemodell 1, 2 zugeführt, das daraus Laser-Bestrahlungsparameter ermittelt und diese an eine Behandlungseinrichtung 20 sendet. Diese kann an demselben Ort angeordnet sein wie die lokalen Einrichtungen 16, 17, 18, 19, so dass der Patient 5 nach der Erfassung seiner aktuellen Zustandsdaten an demselben Ort und grundsätzlich auch mit derselben Einrichtung, mit der die aktuellen Netzhautaufnahmen erstellt worden sind, behandelt werden kann. Dies hat den Vorteil, dass die bildgebenden Verfahren, die für die Netzhautaufnahmen verwendet worden sind, in manchen Fällen auch für die Steuerung, Ausrichtung und Lenkung des Behandlungslasers verwendet werden können. Beispielsweise kann der Behandlungslaser dieselben optischen Elemente, beispielsweise Blenden und Linsen, passieren, die für die Aufnahmen von aktuellen Zustandsdaten genutzt werden. Auch eine Einrichtung 21 zum Eye-tracking kann vorgesehen und auch mit wenigstens einer der Einrichtungen 18, 19 verbunden sein, was durch den Pfeil 23 angedeutet ist. Diese Einrichtung 21 erfasst Augenbewegungen und führt in an sich bekannter Weise den Behandlungslaser 22 geeignet nach.

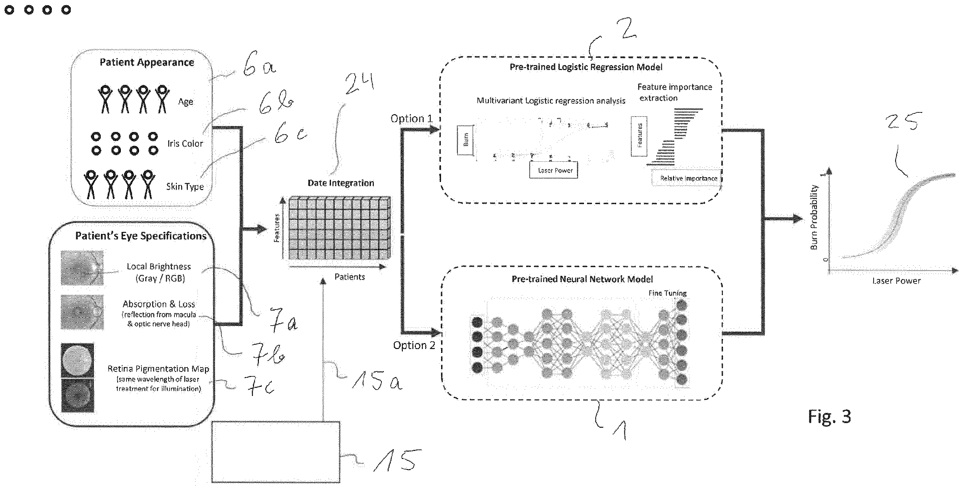

Die Figur 3 zeigt noch in einer detaillierteren Darstellung die Erstellung des Vorhersagemodells 1, 2 mit Trainingsdaten 6a, 6b, 6c, 7a, 7b, 15a, die in einem Integrationsschritt 24 zusammengefasst und zum Training dem Vorhersagemodell zugeführt werden. Das Ergebnis des Vorhersagemodells ist danach bei einer Analyse eines einzelnen Patienten eine "Risikokurve" 25, der die Laserbestrahlungsparameter 10a, 10b, 10c in Abhängigkeit von der gewünschten oder zulässigen Wahrscheinlichkeit einer Gewebeschädigung/Veränderung entnommen werden können. Vor der Extraktion von konkreten Laser-Bestrahlungsparametern aus der Risikokurve 25 für einen Behandlungsfall kann optional noch ein Freigabeschritt 26 vorgesehen werden, der in der Figur 2 dargestellt ist und beispielsweise durch einen Augenarzt vorgenommen werden kann.

Mit dem beschriebenen System ist eine einfache und wenig aufwändige, dabei jedoch sichere und nicht gesundheitsgefährdende Netzhautbehandlung von Patienten ermöglicht.

## Patentansprüche

1. Verfahren zum Ausbilden eines Vorhersagemodells (1, 2), insbesondere zum Trainieren eines neuronalen Netzes (1) oder eines Algorithmus (2), oder zur Gewinnung von Daten für eine Regressionsanalyse zur Bildung einer Zuordnungsfunktion, wobei das Vorhersagemodell, insbesondere das neuronale Netz, der Algorithmus oder die Zuordnungsfunktion, aktuellen Zustandsparametern (3a, 3b, 3c, 4a, 4b, 4c) eines Patienten (5) einen oder mehrere Laser-Bestrahlungsparameter (10a, 10b, 10c) für eine Netzhautbehandlung zuordnet, **dadurch gekennzeichnet, dass** als Trainingsdaten für eine Mehrzahl von Patienten (9a, 9b, 9c) jeweils einerseits als Trainings-Eingangsparameter wenigstens eine durch ein bildgebendes Verfahren gewonnene Netzhautaufnahme (7a, 7b, 7c) des Patienten, und andererseits als Trainings-Ergebnisparameter ein Schwellwert für den/die Laser-Bestrahlungsparameter (8a, 8b, 8c), bei dessen/deren Überschreitung bleibende schädigende Gewebeveränderungen eintreten, verwendet werden, und wobei insbesondere zusätzlich als Trainings-Eingangsparameter wenigstens ein unabhängig von der Netzhaut des Patienten ermittelter Parameter (6a, 6b, 6c), insbesondere ein Hautparameter, ein Haarparameter, ein Irisparameter, das Alter des Patienten, eine physiologisch wirksame Lebensgewohnheit oder eine Vorerkrankung, verwendet wird.

2. Verfahren nach Anspruch 1, **dadurch gekennzeichnet, dass** zum Trainieren für jeden Patienten wenigstens zwei Netzhautaufnahmen (7a, 7b, 7c) als Trainings-Eingangsparameter verwendet werden, die die zu unterschiedlichen Zeitpunkten, insbesondere vor und nach einer Titration, und/oder mit verschiedenen bildgebenden Verfahren und/oder bei unterschiedlichen technischen Aufnahmebedingungen gewonnen werden.

3. Verfahren nach Anspruch 1 oder 2, **dadurch gekennzeichnet, dass** als bildgebende Verfahren für die Ermittlung der Trainings-Eingangsparameter (7a, 7b, 7c) eines oder zwei der folgenden Verfahren verwendet werden:
Fotografische Farbbildaufnahme, fotografische Infrarotaufnahme, OCT-B-scan, ein Tiefenschnittbild einer mit optischer Kohärenztomografie erstellten Aufnahme, BAF, eine Fluoreszenzaufnahme ohne zusätzlichen Farbstoff mit einer Anregungswellenlänge 488nm, Scanning laser ophtalmoscopy, OCT in Verbindung mit Scanning laser ophtalmoscopy, FAG, eine Fluoreszenzaufnahme mit Farbstoff Fluorescein bei einer Anregungswellenlänge 488nm.

4. Verfahren nach einem der Ansprüche 1 bis 3, **dadurch gekennzeichnet, dass** der jeweilige Schwellwert (8a, 8b, 8c) für den/die Laser-Bestrahlungsparameter für jeden Patienten (9a, 9b, 9c) durch eine Reihe von Titrationsschüssen auf die Netzhaut des Patienten mit verschiedenen Laser-Bestrahlungsparametern und einen nachfolgenden Nachweis von Gewebeveränderungen anhand von jeweils mehreren, durch verschiedene bildgebende Verfahren gewonnenen Netzhautabbildungen ermittelt wird, wobei insbesondere jeweils der geringste mit einem der bildgebenden Verfahren ermittelte Schwellwert als Trainings- Ergebnisparameter für das Ausbilden des Vorhersagemodells, insbesondere das Training eines neuronalen Netzes, verwendet wird.

5. Verfahren nach einem der Ansprüche 1 bis 3, **dadurch gekennzeichnet, dass** der jeweilige Schwellwert (8a, 8b, 8c) für den/die Laser-Bestrahlungsparameter für jeden Patienten (9a, 9b, 9c) durch eine Reihe von Titrationsschüssen auf die Netzhaut des Patienten mit verschiedenen Laser-Bestrahlungsparametern und einen nachfolgenden Nachweis von Gewebeveränderungen anhand von jeweils mehreren, durch verschiedene bildgebende Verfahren gewonnenen Netzhautabbildungen ermittelt wird, wobei als Trainings-Ergebnisparameter für das Ausbilden des Vorhersagemodells, insbesondere das Training eines neuronalen Netzes, für alle untersuchten Patienten der durch dasjenige bildgebende Verfahren ermittelte Schwellwert verwendet wird, welches bei den meisten untersuchten Patienten den geringsten Schwellwert anzeigt.

6. Verfahren nach einem der Ansprüche 1 bis 3, **dadurch gekennzeichnet, dass** der jeweilige Schwellwert (8a, 8b, 8c) für den/die Laser-Bestrahlungsparameter für jeden Patienten (9a, 9b, 9c) als Trainings-Ergebnisparameter durch eine Reihe von Titrationsschüssen auf die Netzhaut des Patienten mit verschiedenen Laser-Bestrahlungsparametern und einen nachfolgenden Nachweis von Gewebeveränderungen anhand der optischen Kohärenztomografie in Verbindung mit IR-scanning Laser- Ophtalmoskopie ermittelt wird.

7. Verfahren nach einem der Ansprüche 1 bis 6, **dadurch gekennzeichnet, dass** als Laser-Bestrahlungsparameter, der bei den Titrationsschüssen variiert und dessen Schwellwert (8a, 8b, 8c) als Trainings-Ergebnisparameter ermittelt wird, eine Leistung des Bestrahlungslasers oder eine Leistungsdichte des Laserspots bei ansonsten festgelegten Laser-Bestrahlungsparametern oder eine über die Bestrahlungszeit insgesamt deponierte Energie auf einem Laserspot festgelegter Größe gewählt wird.

8. Verfahren nach einem der Ansprüche 1 bis 7, **dadurch gekennzeichnet, dass** ein Hautparameter und/oder ein Haarparameter und/oder ein Irisparameter (6a, 6b, 6c) durch ein optisches Messverfahren ermittelt wird, wobei insbesondere eine Farbe und/oder eine Reflektivität der Haut, des Haars oder der Iris eines Patienten gemessen wird.

9. Vorhersagemodell, das aktuellen Zustandsparametern (3a, 3b, 3c, 4a, 4b, 4c) eines Patienten (5) einen oder mehrere Laser-Bestrahlungsparameter (10a, 10b, 10c) für eine Netzhautbehandlung zuordnet und das nach einem der Ansprüche 1 bis 8, insbesondere durch Trainieren eines neuronalen Netzes (1) oder eine Regressionsanalyse und Bildung einer Zuordnungsfunktion, ausgebildet ist.

10. Verfahren zur Ermittlung von Laser-Bestrahlungsparametern (10a, 10b, 10c) zur Behandlung der Netzhaut eines Patienten (5), **dadurch gekennzeichnet, dass**
In einem ersten Schritt Zustandsparameter (3a, 3b, 3c, 4a, 4b, 4c) des Patienten ermittelt werden und dass in einem weiteren Schritt den Zustandsparametern durch ein Vorhersagemodell (1, 2) gemäß Anspruch 9 ein oder mehrere Laser-Bestrahlungsparameter (10a, 10b, 10c) für eine Netzhautbehandlung zugeordnet werden, wobei die Zustandsparameter eine, zwei oder mehr als zwei jeweils durch ein bildgebendes Verfahren gewonnene Netzhautaufnahmen (4a, 4b, 4c) des Patienten (5) umfassen, wobei im Fall von mehr als einer Netzhautaufnahme die Netzhautaufnahmen mit verschiedenen bildgebenden Verfahren und/oder unter verschiedenen Aufnahmebedingungen gewonnen werden.

11. Verfahren zur Ermittlung von Laser-Bestrahlungsparametern (10a, 10b, 10c) nach Anspruch 10, **dadurch gekennzeichnet, dass** die Zustandsparameter (3a, 3b, 3c, 4a, 4b, 4c) einen oder mehrere der folgenden Parameter umfassen: einen Hautparameter, einen Haarparameter, einen Irisparameter, das Alter des Patienten, eine physiologisch wirksame Lebensgewohnheit, eine Vorerkrankung.

12. Verfahren nach Anspruch 11, **dadurch gekennzeichnet, dass** ein Hautparameter und/oder ein Haarparameter und/oder ein Irisparameter (3a, 3b, 3c) des Patienten (5) durch ein optisches Messverfahren ermittelt wird, wobei insbesondere eine Farbe und/oder eine Reflektivität der Haut, des Haars oder der Iris des Patienten gemessen wird und/oder zur Bestimmung eines Zustandsparameters (3a, 3b, 3c) jeweils eine fotografische Aufnahme der Haut, der Haare oder der Iris des Patienten durch das Vorhersagemodell ausgewertet wird.

13. Verfahren nach Anspruch 10, 11 oder 12, **dadurch gekennzeichnet, dass** aus wenigstens einer Netzhautaufnahme (4a, 4b, 4c) des Patienten Bereiche ermittelt und für eine nachfolgende Laserbehandlung von der Bestrahlung ausgenommen werden, deren Reflektivität von einer Durchschnittsreflektivität der Netzhaut um wenigstens 50% abweicht, insbesondere mehr als 50% höher ist als die Durchschnittsreflektivität der Netzhaut.

14. Verfahren nach Anspruch 10, 11, 12 oder 13, **dadurch gekennzeichnet, dass** aus der Netzhautaufnahme (4a, 4b, 4c) des Patienten die Varianz der Reflektivitätswerte, deren Differenz von der Durchschnittsreflektivität um weniger als 50 % differieren, ermittelt und aus der Varianz ein Korrekturwert für die durch das Vorhersagemodell (1, 2) ermittelten Laser-Bestrahlungsparameter (10a, 10b, 10c) ermittelt wird und/oder, dass aus der auf dem Nervenkopf ermittelten Reflektivität ein Verlustparameter ermittelt wird, der die Verluste durch Absorption und Streuung von Licht im Glaskörper des Auges beschreibt und dass dieser Verlustparameter bei der Bestimmung der Laserbestrahlungsparameter (10a, 10b, 10c) berücksichtigt wird.

15. Einrichtung zur Netzhautbehandlung von Patienten mittels Laserbestrahlung mit wenigstens einem Behandlungslaser (22) sowie wenigstens einer Vorrichtung (18, 19) zur Erstellung einer Netzhautaufnahme und mit einer Verarbeitungseinrichtung mit einem Vorhersagemodell (1, 2) nach Anspruch 9, die wenigstens einer aktuell durch die Vorrichtung erstellten Netzhautaufnahme (4a, 4b, 4c) und insbesondere einem zusätzlichen, aktuell ermittelten Zustandsparameter (3a, 3b, 3c) einen oder mehrere Laser-Bestrahlungsparameter (10a, 10b, 10c) zur Steuerung für den Behandlungslaser zuordnet.
